**Europäisches Patentamt**

**European Patent Office**

**Office européen des brevets**

(19)

(11) Veröffentlichungsnummer: **0 149 280**
**B1**

(12) # EUROPÄISCHE PATENTSCHRIFT

(45) Veröffentlichungstag der Patentschrift:
**09.03.88**

(51) Int. Cl.⁴: **H 04 B 9/00**

(21) Anmeldenummer: **84201910.1**

(22) Anmeldetag: **19.12.84**

(54) **Anordnung zur Datenübertragung zwischen zwei relativ zueinander drehbaren Teilen.**

(30) Priorität: **07.01.84 DE 3400361**

(43) Veröffentlichungstag der Anmeldung:
**24.07.85 Patentblatt 85/30**

(45) Bekanntmachung des Hinweises auf die Patenterteilung:
**09.03.88 Patentblatt 88/10**

(84) Benannte Vertragsstaaten:
**DE FR GB IT NL SE**

(56) Entgegenhaltungen:
**EP - A - 0 039 475**
**DE - A - 2 558 557**
**FR - A - 2 449 994**
**FR - A - 2 521 751**
**GB - A - 1 204 296**
**US - A - 3 448 274**
**US - A - 4 373 207**

(73) Patentinhaber: **Philips Patentverwaltung GmbH,**
**Wendenstrasse 35 Postfach 10 51 49,**
**D-2000 Hamburg 1 (DE)**

(84) Benannte Vertragsstaaten: **DE**

(73) Patentinhaber: **N.V. Philips' Gloeilampenfabrieken,**
**Groenewoudseweg 1, NL-5621 BA Eindhoven (NL)**

(84) Benannte Vertragsstaaten: **FR GB IT NL SE**

(72) Erfinder: **Helzel, Thomas, Oldesloer Strasse 107,**
**D-2000 Hamburg 61 (DE)**

(74) Vertreter: **Hartmann, Heinrich, Dipl.-Ing. et al, Philips**
**Patentverwaltung GmbH**
**Wendenstrasse 35 Postfach 10 51 49,**
**D-2000 Hamburg 1 (DE)**

ACTORUM AG

## Beschreibung

Die Erfindung betrifft eine Anordnung zur Datenübertragung zwischen zwei relativ zueinander drehbaren Teilen, von denen der eine eine Sendeanordnung und der andere eine Empfängeranordnung trägt, wobei die Empfängeranordnung wenigstens zwei Empfangswandler umfasst, die Signale empfangen, deren Phasenlage und/oder Amplituden sich beim Drehen der Teile gegensinnig ändert.

Eine solche Anordnung ist aus der DE-A-32 05 065 bekannt und in Fig. 1 dargestellt. Die bekannte Anordnung umfasst einen hohlzylindrischen und auf seiner Innenseite reflektierenden Spiegel 3, mit dem eine Sendeanordnung in Form eines Lasers 4 fest verbunden ist. Ein nicht näher dargestellter, im Innern des Spiegels 3 angeordneter Teil trägt eine aus zwei Empfangswandlern (Fotoelementen) 10 und 20 bestehende Empfängeranordnung. Der Spiegel 3 und der die Empfängeranordnung tragende Teil sind relativ zueinander drehbar.

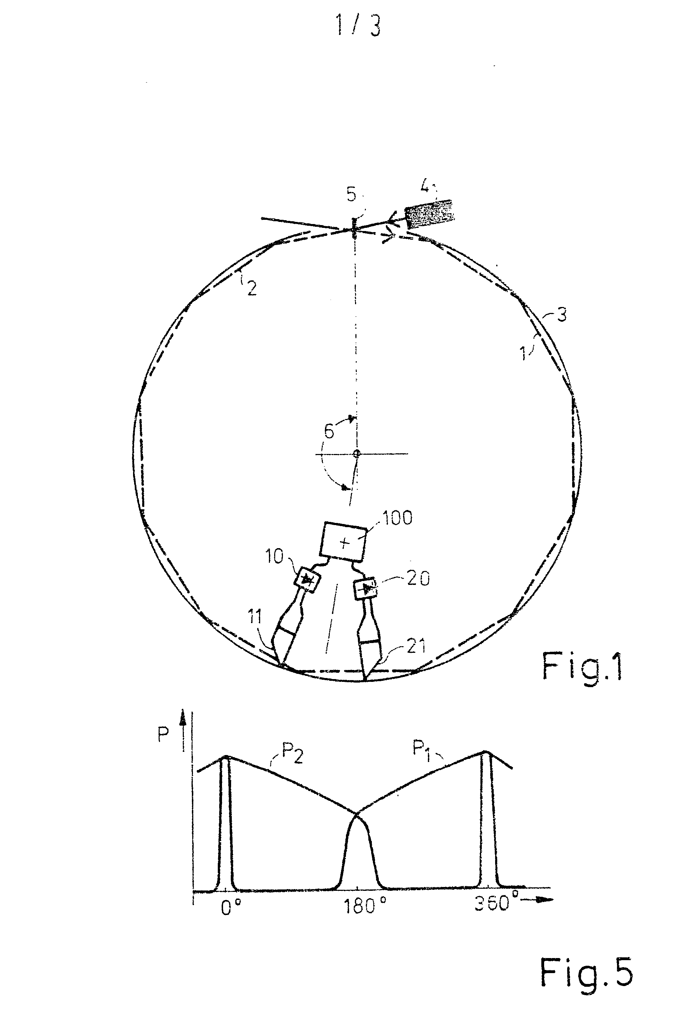

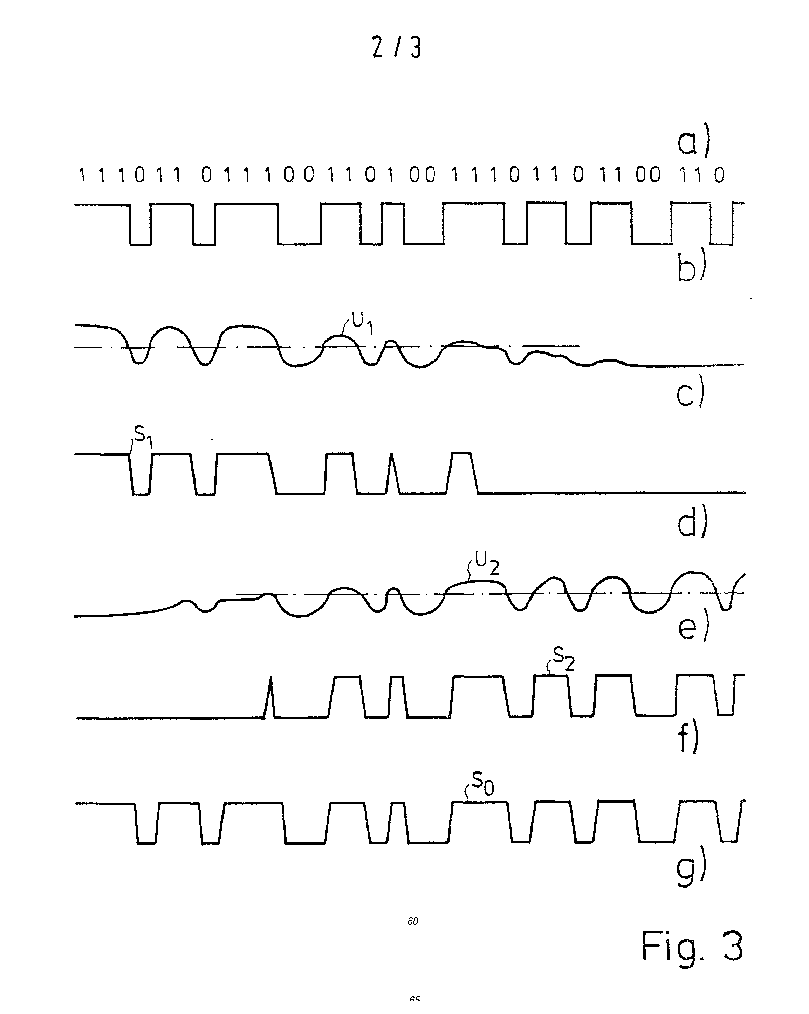

Der Laser 4 sendet ein paralleles Strahlenbündel aus, das durch einen Strahlteiler 5 in Form eines Spiegels in zwei senkrecht zur Zeichenebene der Fig. 1 versetzte Strahlenbündel 1 und 2 aufgeteilt wird, die mit gleicher Intensität, jedoch entgegengesetztem Umlaufsinn und in senkrecht zur Zeichenebene der Fig. 1 versetzten Ebenen auf der Innenfläche des Spiegels 3 reflektiert werden. Diese Aufteilung in zwei Strahlenbündel ist erforderlich, weil die Bitrate (jedes Bit ist durch eine hohe oder eine niedrige Intensität des Laserlichtes gekennzeichnet) so gross ist, dass sich andernfalls bei der Drehung der Teile Laufzeitsprünge einstellen würden, die die Auswertung des Signals unmöglich machen würden. In den Ebenen, in denen die Strahlen 1 bzw. 2 umlaufen, ist jeweils etwa eine Hälfte der Innenfläche des Hohlzylinders 3 nicht reflektierend, so dass sich etwa folgendes Verhalten ergibt:

Solange – wie in Fig. 1 dargestellt – der Winkel 6 zwischen dem Strahlteiler 5 und der Mitte der Empfangsanordnung deutlich kleiner ist als 180° wird das Strahlenbündel 2 über den Spiegel 21 dem Empfangswandler 20 zugeführt. Der Empfangswandler 10 empfängt in dieser Winkelstellung kein Signal, weil der Strahl 1, bevor er den dem Fotoelement 10 zugeordneten Spiegel 11 erreichen kann, auf den nichtreflektierenden Teil der Innenfläche trifft, wo er absorbiert wird. Wird der Winkel 6 deutlich über 180° hinaus vergrössert, liegen die Verhältnisse umgekehrt. Das Strahlenbündel wird dann dem Fotoelement 10 zugeleitet, während das Fotoelement 20 kein Licht empfängt. – Wenn die Anordnung 10, 20 dem Spiegel 5 diametral gegenübersteht (der Winkel 6 beträgt dann gerade 180°) und in einem gewissen Winkelbereich (z.B. 2°) um diese Winkelstellung herum empfangen beide Fotoelemente 10, 20 die zugeordneten Strahlenbündel 1 und 2.

Zur weiteren Auswertung sind die beiden Fotoelemente bei der bekannten Anordnung parallelgeschaltet (vgl. Seite 4, 2. Absatz der DE-OS 32 05 065), d.h. die Ströme der Fotodioden werden von einer Addierschaltung 100 addiert. Durch diese Addition werden auch die mit beiden Signalen verknüpften Rauschpegel addiert – und zwar auch dann, wenn eines der Fotoelemente nicht von dem zugeordneten Strahlenbündel getroffen werden kann, was zur Folge hat, dass die Eingangsempfindlichkeit der Auswerteschaltung verringert wird.

Aufgabe der Erfindung ist es, ohne eine derartige Verringerung der Eingangsempfindlichkeit aus den Ausgangssignalen der Empfangswandler ein Signal zu erzeugen, das dem Signal der Sendeanordnung weitgehend entspricht.

Diese Aufgabe wird erfindungsgemäss dadurch gelöst, dass den Empfangswandlern je ein Begrenzer-Verstärker nachgeschaltet ist, deren Ausgangssignale den Eingängen einer ODER-Schaltung zugeführt ist, deren Ausgangssignal das Ausgangssignal der Empfängeranordnung bildet.

Durch den Begrenzer-Verstärker erhalten alle Momentanwerte des Signals, die oberhalb eines bestimmten Schwellwertes liegen, einen ersten Pegel und alle darunter liegenden Momentanwerte einen zweiten Pegel, so dass das Signal – wenn es überhaupt den Schwellenwert überschreitet – in ein im wesentlichen binäres Signal umgesetzt wird, das durch die ODER-Schaltung weiterverarbeitet wird. Dabei werden die in den beiden Kanälen vorhandenen Rauschpegel nicht addiert.

Die Erfindung wird nachstehend anhand der Zeichnung näher erläutert. Es zeigen

Fig. 1 die bekannte Anordnung zur Datenübertragung, bei der auch die Erfindung anwendbar ist,

Fig. 2 eine erste Ausführungsform der Erfindung,

Fig. 3 den zeitlichen Verlauf der Signale in den verschiedenen Kanälen und am Ausgang der ODER-Schaltung,

Fig. 4 eine weitere Ausführungsform,

Fig. 5 den Signalpegel in den beiden Kanälen als Funktion des Drehwinkels 6.

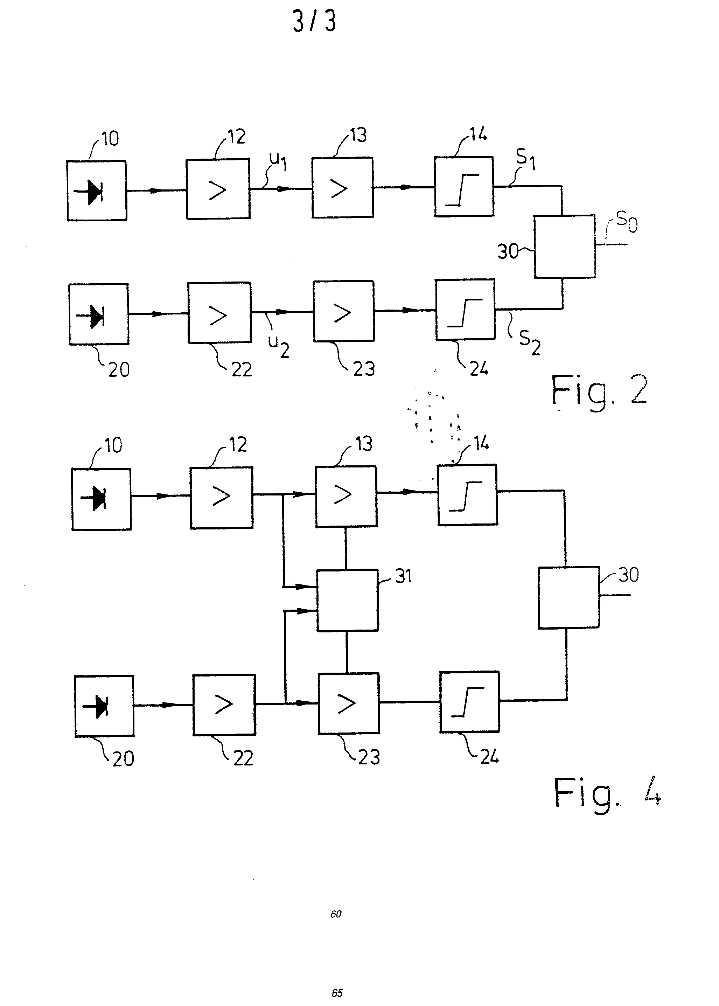

Die von den beiden Empfangswandlern, den Fotodioden 10 und 20, die ebenso wie der Sender auf einer Anordnung nach Fig. 1 angeordnet sind, gelieferten Signale werden je einem Vorverstärker 12 bzw. 22 zugeführt (Fig. 2). Das Ausgangssignal $u_1$ bzw. $u_2$ der Vorverstärker 12 bzw. 22 wird einem Hochfrequenzverstärker 13 bzw. 23 zugeführt, der entweder oder geregelter (breitbandiger) Gleichspannungsverstärker oder als Wechselspannungsverstärker ausgebildet ist. Das Ausgangssignal des HF-Verstärkers 13 bzw. 23 wird einem Begrenzer-Verstärker 14 bzw. 24 zugeführt. Die Ausgangssignale $s_1$ und $s_2$ der Begrenzer-Verstärker 14 und 24 werden den Eingängen einer ODER-Schaltung 30 zugeführt, deren Ausgangssignal $s_0$ im wesentlichen den gleichen zeitlichen Verlauf hat wie die Intensität der Strahlung des Lasers 4. Die von der Anordnung zur Datenübertragung zu übertragende Information ist in Fig. 3a dargestellt. Diese Information besteht aus einer Folge von «1» und «0». Die Bitrate beträge etwa 150 Mbit/s, wenn die Anordnung zur Datenübertragung bei einem Computertomographen eingesetzt wird.

Die Information gemäss Fig. 3a steuert die Intensität des Lasers derart, dass die vom Laser emittierten Strahlenbündel 1, 2 bei einer «1» eine hohe Intensität und bei einer «0» keine bzw. eine sehr geringe Intensität aufweisen. Der zeitliche Verlauf der Intensität der Strahlenbündel 1, 2 ist in Fig. 3b dargestellt.

Eines der beiden Strahlenbündel 1, 2 erreicht den zugeordneten Empfangswandler 10 bzw. 20; wenn der Winkel zwischen dem Trennspiegel 5 und der Empfangsanordnung 10, 20 180° beträgt, erreichen beide Strahlenbündel die zugehörigen Empfangswandler. Laufzeit und Amplitude hängen dabei vom Winkel 6 ab. Für die Zwecke der Computertomographie beträgt der Durchmesser des Spiegels 3 etwa 1 m, so dass die Laufzeit der Lichtstrahlenbündel 1, 2 vom Trennspiegel 5 bis zu der diesem Trennspiegel diametral gegenüberliegenden Stelle auf dem Zylinderspiegel 3 in der Grösse der Taktzeit liegen, mit der die einzelnen Informationsbits aufeinander folgen.

Die von den als Empfangswandler wirksamen Fotodioden 10, 20 beim Empfang der Strahlenbündel 1 und 2 erzeugten elektrischen Signale sind sehr schwach (in der Grössenordnung von mA) und ihr zeitlicher Verlauf ist gegenüber dem zeitlichen Verlauf der Lichtintensität am Ausgang des Lasers (Fig. 3d) erheblich verformt. Durch die Verstärker 12 und 13 bzw. 22 und 23 wird das Ausgangssignal der Empfangswandler 10, 20 linear verstärkt, so dass die Signale an den Ausgängen von 10, 12 und 13 einerseits sowie 20, 22 und 23 andererseits jeweils den gleichen zeitlichen Verlauf aufweisen – jedoch nicht die gleiche Amplitude. Die Signale $u_1$ bzw. $u_2$ in den beiden Kanälen sind in Fig. 3c und 3e dargestellt. Man erkennt, dass die Amplitude des Signals $u_1$ abnimmt, während die Amplitude des Signals $u_2$ zunimmt, weil der die Empfangsanordnung 10, 20 tragende Teil und der Zylinderspiegel 3 relativ zueinander so gedreht werden (mit einer Umdrehung pro Sekunde), dass der Winkel 6 (Fig. 1) abnimmt.

Es ist weiterhin ersichtlich, dass in einem bestimmten zeitlichen (Überlappungs-) Bereich keines der beiden Signale verschwindet. Vor und hinter diesem Bereich ist nur eines der beiden Signale $u_1$ und $u_2$ von Null verschieden, weil die Empfangsanordnung 10, 20 dann eine Winkelstellung einnimmt, in der nur eines der beiden Strahlenbündel 1, 2 den zugeordneten Empfangswandler erreicht, während das andere Strahlenbündel, wie bereits erwähnt, durch die nicht reflektierenden Bereiche des Zylinderspiegels 3 im wesentlichen absorbiert wird. Dieser Bereich ist in der Zeichnung nur wenige Bitperioden lang; in Wirklichkeit beträgt er jedoch etwa 1 Million Bitperioden, wenn man davon ausgeht, dass bei der Drehung mit einer Umdrehung pro Sekunde während etwa 6 msec (entsprechend einem Winkelüberlappungsbereich von rund 2°) beide Empfangswandler ein von Null verschiedenes Signal erhalten. Durch die Erfindung wird dabei ein gleitender Übergang vom einen Kanal zum anderen Kanal erreicht.

In Fig. 3c bzw. Fig. 3e ist als strichpunktierte Linie der Schwellenwert der Begrenzer-Verstärker 14, 24 eingetragen. Wenn die Spannung $u_1$, $u_2$ (nach Verstärkung durch die Verstärker 13, 23) oberhalb dieses Schwellenwertes liegt, nimmt das Ausgangssignal des Begrenzer-Verstärkers 14 bzw. 24 einen ersten Wert an und wenn das Signal darunter liegt einen zweiten Wert. Die so erzeugten Signale $s_1$ und $s_2$ sind in den Figuren 3d und 3f dargestellt. Es ist erkennbar, dass die beiden ersten positiven Impulse des Signals gemäss Fig. 3b nur im Ausgangssignal s des Begrenzer-Verstärkers 14 enthalten sind. Bei dem nächsten Impuls spricht zwar auch schon der Begrenzer-Verstärker 24 an, jedoch nur kurzzeitig, weil das Signal $u_2$ nur kurzzeitig den Schwellenwert erreicht. Dieser Impuls ist wesentlich kürzer als der dritte Impuls des Lasers (Fig. 3b), der jedoch durch den dritten Impuls des Signals $s_1$ recht gut angenähert wird. Der zeitliche Verlauf des Ausgangssignals $s_0$ der ODER-Schaltung entspricht in diesem Zeitpunkt praktisch dem Signal $s_1$, d.h. das schwächere Signal (in diesem Fall $s_2$) wird unterdrückt. Im weiteren Verlauf nimmt die Amplitude von $u_1$ so weit ab und die Amplitude von $u_2$ so weit zu, dass $u_2$ grösser wird als $u_1$. Dann wird der Verlauf des Signals b besser durch $s_2$ als durch $s_1$ wiedergegeben und das Ausgangssignal $s_0$ wird durch die entsprechenden Impulse im Signal $s_2$ bestimmt. Auf diese Weise erfolgt ein allmählicher Übergang von einem Kanal (10, 12, 13, 14) zum anderen Kanal (20, 22, 23, 24), wobei das Ausgangssignal der ODER-Schaltung $s_0$ jeweils durch diejenigen Teile der Signale $s_1$, $s_2$ bestimmt wird, die dem Verlauf des Originalsignals (Fig. 3b) besser entsprechen.

In Fig. 4 ist eine Ausführungsform dargestellt, bei der die Verstärkung in den beiden Kanälen in Abhängigkeit von dem Signalpegel umgeschaltet wird derart, dass die Verstärkung immer in dem Kanal grösser ist, der auch den höheren Signalpegel aufweist. Dazu sei zunächst auf Fig. 5 verwiesen, die die optische Leistung $P_1$, $P_2$ am Ort des Empfangswandlers 10 bzw. 20 als Funktion des Winkels 6 darstellt. Die optische Leistung $P_2$ am Eingang des Empfangswandlers 20 hat bei 0° ein Maximum, weil dabei das Strahlenbündel 2 praktisch ungeschwächt zum Empfangswandler 20 gelangt. Mit zunehmendem Drehwinkel nimmt die Intensität $P_2$ ab, weil das Strahlenbündel 2 reflektiert wird und an Intensität verliert. Wenn der Winkel 6 etwas grösser wird als 180°, ergibt sich eine starke Leistungsabnahme, weil das Strahlenbündel 2 auf seinem Weg zum Fotowandler 20 auf nicht oder nur wenig reflektierende Bereiche trifft, bis bei weiter zunehmendem Drehwinkel 6 die Leistung den Wert $P_0$ erreicht. Die vom Wandler 10 empfangene optische Leistung verläuft als Funktion des Drehwinkels 6 gerade umgekehrt, d.h. symmetrisch zu einer Senkrechten bei 180°.

Bei Winkeln, die etwas grösser sind als 0 bis zu Winkeln, die etwas kleiner sind als 180°, erreicht praktisch kein Licht den Wandler 10. Danach steigt – bis 180° stark und anschliessend geringer – die optische Leistung mit dem Drehwinkel, bis sie bei 360° ein Maximum erreicht. Somit können die optischen Leistungen, die von den Wandlern 10 und 20 empfangen werden, bzw. das Verhältnis dieser Leistungen als Mass für die Winkelstellung benutzt werden, um im Bereich von 180° einen Übergang von dem einen Kanal auf den anderen Kanal zu erreichen. Grundsätzlich könnte dies auch mit Hilfe eines Winkelgebers folgen, doch wäre dies ein zusätzlicher Aufwand.

Da der zeitliche Mittelwert der Signale $u_1$, $u_2$ (gemittelt über eine im Vergleich zu einer vollen Umdrehung zwischen Sendeanordnung 4 und Empfängeranordnung 10, 2 kurzen Zeitraum) der optischen Leistung $P_1$, $P_2$ entspricht, können auch die Signale $u_1$, $u_2$ zur Steuerung der Verstärker herangezogen werden. Zu diesem Zweck werden diese Signale vom Ausgang der Vorverstärker 12 bzw. 22 über ein nicht näher dargestelltes, geeignet bemessenes Zeitkonstantenglied den Eingängen eines Fensterkomparators 31 zugeführt, der mit Steuereingängen der Hochfrequenzverstärker 13 und 23 gekoppelt ist, und zwar derart, dass beide Hochfrequenzverstärker die gleiche Empfindlichkeit aufweisen, wenn die Pegeldifferenz zwischen beiden Eingangssignalen einen vorgegebenen Wert (z.B. 5dB) unterschreitet und anderenfalls die Empfindlichkeit desjenigen HF-Verstärkers 13 bzw. 23 herabgesetzt wird, dem das Signal mit dem kleineren Pegel zugeführt wird. Die Empfindlichkeitsänderung kann in Form einer Änderung der Verstärkung oder durch ein Verschieben des Arbeitspunktes (Triggerpegel) erfolgen.

Der Fensterkomparator kann aus zwei Komparatoren aufgebaut sein, denen das eine der beiden Signale $u_1$ bzw. $u_2$ direkt und das andere ($u_2$ bzw. $u_1$) über ein Dämpfungsglied zugeführt wird, dessen Dämpfung gleich dem erwünschten Pegelunterschied (5 dB) ist, und deren Ausgänge mit den Steuereingängen der Hochfrequenzverstärker 13 bzw. 23 verbunden ist. Das Ausgangssignal der in der Empfindlichkeit steuerbaren Hochfrequenzverstärker 13 bzw. 23 wird wiederum durch je einen Begrenzer-Verstärker 14 bzw. 24 umgeformt und den beiden Eingängen der ODER-Schaltung 30 zugeführt, so dass sich die Schaltung nach Fig. 4 von der Schaltung nach Fig. 2 nur durch den Fensterkomparator 31 und dadurch unterscheidet, dass die Hochfrequenzverstärker 13 bzw. 23 eine durch ein Signal an ihrem Steuereingang steuerbare Empfindlichkeit aufweisen. Durch diese Modifikation wird erreicht, dass Störsignale, in dem jeweils für die Datenübertragung nicht wirksamen Kanal auch dann unterdrückt werden, wenn ihre Amplitude in der Grössenordnung des normalen Signalpegels liegt.

Obwohl die Erfindung anhand einer Datenübertragungsanordnung gemäss der DE-OS 32 05 065 erläutert wurde, ist bei anderen Anordnungen zur Datenübertragung zwischen zwei relativ zueinander drehbaren Teilen anwendbar, bei denen sich die Phasenlage (Laufzeit) und/oder die Amplitude des empfangenen Signals bei einer Drehung zwischen den Teilen ändert. Ein Beispiel hierfür ergibt sich aus der DE-A-33 31 722, (veröffentlicht am 21.3.85), wenn dabei ein zweites Empfänger-Koppelelement benutzt wird, das so angeordnet ist, dass es der Mitte zwischen zwei benachbarten Sender-Koppelelementen gegenübersteht, wenn das andere Empfänger-Koppelelement genau einem Sender- Koppelelement gegenübersteht. Dabei ist die von dem einen Koppelelement empfangene Amplitude maximal, während die vom anderen Koppelelement empfangene Signalamplitude minimal ist und auch hier ist es möglich, in der vorstehend beschriebenen Weise vom einen Kanal auf den anderen Kanal überzugehen, so dass immer nur derjenige Kanal mit dem jeweils stärksten Signal die Datenübertragung bestimmt.

**Patentansprüche**

1. Anordnung zur Datenübertragung zwischen zwei relativ zueinander drehbaren Teilen, von denen der eine eine Sendeanordnung (4) und der andere eine Empfängeranordnung trägt, wobei die Empfängeranordnung wenigstens zwei Empfangswandler (10, 20) umfasst, die Signale empfangen, deren Phasenlage und/oder Amplituden sich beim Drehen der Teile gegensinnig ändert, dadurch gekennzeichnet, dass den Empfangswandlern (10, 20) je ein Begrenzer-Verstärker (14, 24) nachgeschaltet ist, deren Ausgangssignale ($s_1$, $s_2$) den Eingängen einer ODER-Schaltung (30) zugeführt ist, deren Ausgangssignal ($s_0$) das Ausgangssignal der Empfängeranordnung bildet.

2. Anordnung nach Anspruch 1, dadurch gekennzeichnet, dass zum Steuern der Verstärker (13, 23) in den Kanälen vom Ausgang der Empfangswandler (10, 20) zu den Eingängen der ODER-Schaltung (30), eine Schaltung (31) vorgesehen ist, die bei Amplituden- und/oder Phasengleichheit die Empfindlichkeit in dem einen Kanal herauf- und/oder im anderen Kanal herabsetzt.

3. Anordnung nach Anspruch 2, dadurch gekennzeichnet, dass die Schaltung (31) einen Komparator enthält, der die Pegel der von den Empfangswandlern (10, 20) gelieferten Signale ($u_1$, $u_2$) vergleicht und die Verstärker (13, 23) der beiden Kanäle so steuert, dass die Verstärkung in dem Kanal mit dem kleinen Pegel kleiner ist als in dem Kanal mit dem grösseren Pegel.

**Revendications**

1 Dispositif pour la transmission de données entre deux parties pouvant tourner l'une par rapport à l'autre, dont l'une porte un dispositif d'émission (4) et l'autre, un dispositif de réception, le dispositif de réception comportant au moins deux transducteurs de réception (10, 20), qui reçoivent des signaux dont les positions de phases et/ou les amplitudes se modifient en sens opposés lorsque les parties tournent, caractérisé en ce que les transducteurs de réception (10, 20) sont suivis chacun d'un amplificateur limiteur (14, 24) dont les signaux de sortie ($s_1$, $s_2$) sont appliqués aux entrées d'un circuit-porte OU (30) dont le signal de sortie ($s_0$) forme le signal de sortie du dispositif de réception.

2. Dispositif suivant la revendication 1, caractérisé en ce que pour commander les amplificateurs (13, 23), dans les canaux allant de la sortie des transducteurs de réception (10, 20) à l'entrée du circuit-porte OU (30) est prévu un circuit qui, en cas d'égalité d'amplitude et/ou de phase, augmente la sensibilité dans le premier canal et/ou la diminue dans l'autre.

3. Dispositif suivant la revendication 2, caractérisé en ce que le circuit (31) contient un comparateur qui compare les niveaux des signaux ($u_1$, $u_2$) fournis par les transducteurs de réception (10, 20) et commande les amplificateurs (13, 23) des deux canaux d'une manière telle que l'amplification dans le canal à niveau peu élevé soit inférieure à celle dans le canal à niveau élevé.

## Claims

1. An arrangement for data transmission between two parts which are rotatable with respect to one another, one of which carries a transmitting arrangement (4) and the other a receiving arrangement, the receiving arrangement comprising at least two reception transducers (10, 20) which receive signals whose phase position and/or amplitudes change in opposite directions upon rotation of the parts, characterized in that each of the reception transducers (10, 20) is followed by a limiter amplifier (14, 24) whose output signals ($s_1$, $s_2$) are applied to the inputs of an OR-circuit (30) whose output signal ($S_0$) forms the output signal of the receiving arrangement.

2. An arrangement as claimed in Claim 1, characterized in that, for driving the amplifiers (13, 23) in the channels from the output of the reception transducers (10, 20) to the inputs of the OR-circuit (30), a circuit (31) is provided which, upon equality in the amplitudes and/or phases, increases the sensitivity in the one channel and/or decreases the sensitivity in the other channel.

3. An arrangement as claimed in Claim 2, characterized in that the circuit (31) contains a comparator which compares the levels of the signals ($u_1$, $u_2$) delivered by the reception transducers (10, 20) and drives the amplifiers (13, 23) of the two channels in such a way that the amplification in the channel with the lower level is lower than in the channel with the higher level.

Fig.1

Fig.5

7

Fig. 3

Fig. 2

Fig. 4

*60*

*65*

11